# EUROPEAN PATENT APPLICATION

(11) **EP 4 047 093 A1**
(43) Date of publication of application: **24.08.2022**
(21) Application number: 20875862.3
(22) Date of filing: 05.10.2020
(51) Int. Cl.: C12N 15/11, C12N 15/09, C12P 19/34, C12P 21/00

(54) **MRNA AND METHOD FOR PRODUCING SAME, AND APPARATUS FOR PRODUCING PROTEIN AND METHOD FOR PRODUCING PROTEIN**

(30) Priority: 15.10.2019 JP 2019188411; 15.07.2020 JP 2020121249
(71) Applicant: Japan Science and Technology Agency, Kawaguchi-shi, Saitama 332-0012 (JP)
(72) Inventor: ABE, Hiroshi, Nagoya-shi, Aichi 464-8601 (JP); ABE, Naoko, Nagoya-shi, Aichi 464-8601 (JP)
(74) Representative: Grünecker Patent- und Rechtsanwälte PartG mbB
(86) International application number: PCT/JP2020/037706
(87) International publication number: WO 2021/075293

(57) **Abstract**

Provided is an mRNA that is used for synthesis of a protein, including a translation region containing a start codon and a stop codon and an untranslated region positioned on the 5'-end side of the start codon, in which some of phosphate groups within the range of at least from the 5' end of the untranslated region to 15 nt on the 3'-end side of the start codon are substituted with phosphorothioate groups. Further provided is a method for producing an mRNA, including: a step of preparing a DNA template; a step of preparing an unmodified NTP containing ATP, GTP, CTP, and UTP, and a modified NTP in which at least one kind of phosphate group of the unmodified NTP is substituted with a phosphorothioate group; and a step of performing transcription reaction with RNA polymerase using the DNA template as a template and the unmodified NTP and the modified NTP as substrates.

## Description

### Technical Field

The present invention relates to an mRNA, a method for producing the mRNA, a device for producing a protein, and a method for producing a protein.

### Background Art

It is known that biological stability can be imparted by introducing a phosphorothioate group (PS) into a nucleic acid. The nucleic acid in which PS has been introduced is used in a molecular structure of an antisense nucleic acid or a siRNA, and is also used in an oligonucleic acid drug on the market. The mRNA modified with PS (hereinafter, may be referred to as "PS-modified") (hereinafter, may be referred to as "PS-mRNA") can be prepared enzymatically by using T7 RNA polymerase or E. coli RNA polymerase and using nucleotide triphosphate substrate (NTPαS) in which PS has been introduced at the α-position of the triphosphate.

Although a method for synthesizing NTPαS has been already developed, PS modification is rarely used for mRNA, and is only used in a few cases reported nearly 30 years ago (for example, see Non Patent Literatures 1 to 3). These reports relate to protein synthesis using a cell-free translation system of E. coli.

Non Patent Literature 1 discloses that PS-mRNA including a poly-U sequence is synthesized, and polyalanine is synthesized with an efficiency of 45% of that of the natural sequence (the third paragraph from the end). In this literature, the sequence is extremely limited, and it cannot be deemed strictly that the product is a protein.

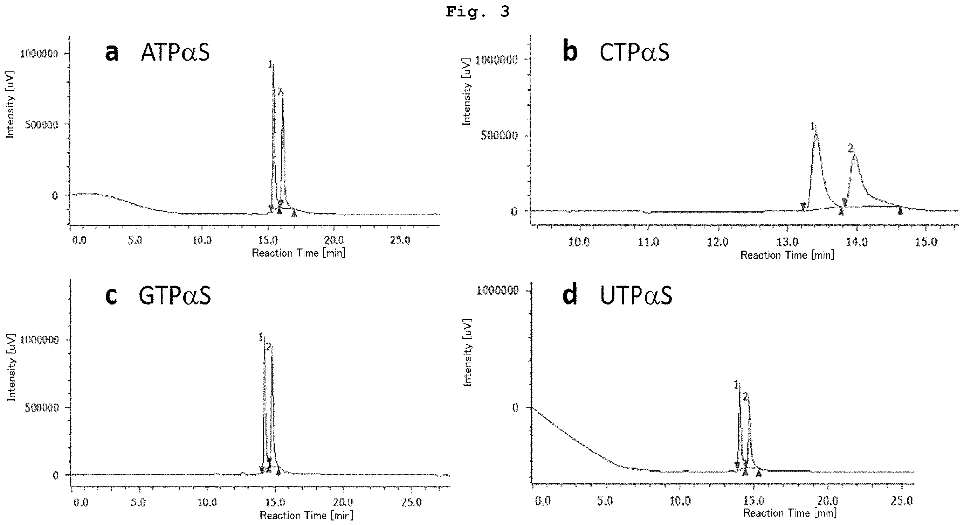

In Non Patent Literature 2, PS-mRNA is prepared by an in vitro transcription method using T7 RNA polymerase with the use of T7 phage DNA as a template. In this literature, the translation activity of PS-mRNA has been evaluated in cell-free translation systems of Thermus thermophilus and E. coli, and it has been reported that PS-mRNA in which one base had been substituted with NTPαS showed up to twice as much protein synthesis as the unsubstituted product in these cell-free systems (Fig. 3) .

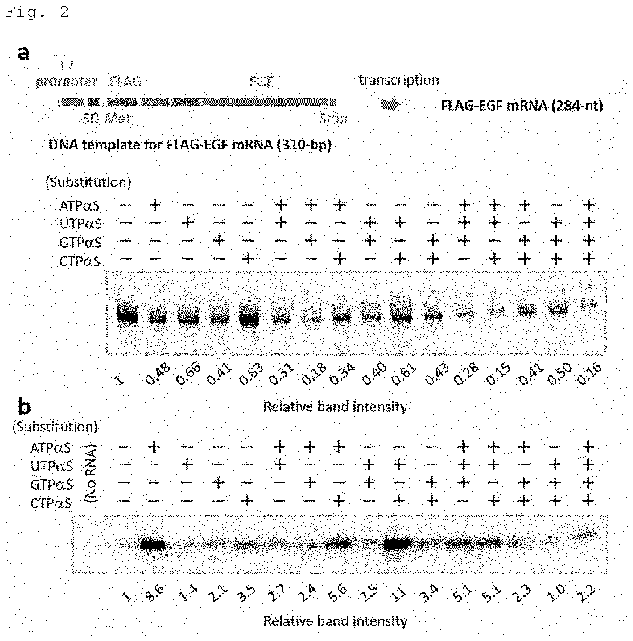

In Non Patent Literature 3, PS-mRNA of E. coli dihydrofolate reductase is prepared by an in vitro transcription method, and the amount of protein synthesis is measured with the use of the PS-mRNA as a template (Fig. 2). In this paper, it has been reported that PS-mRNA in which only one kind of base had been substituted with NTPαS and PS-mRNA in which all the four bases had been substituted with NTPαS during transcription were prepared, and in the former, the protein in an amount around 2 to 3 times that of unsubstituted mRNA (hereinafter, may be referred to as "PO-mRNA") was synthesized. The inventors presume that this improvement in the synthetic efficiency has been brought about by the stability of PS-mRNA. However, in this literature, it has been also reported that the amount of protein synthesis in the PS-mRNA in which all the four bases had been substituted with NTPαS was lower than that of unsubstituted mRNA (around 30% of PO-mRNA).

### Citation List

### Patent Literature

Non Patent Literature 1: Polyribonucleotides containing a phosphorothioate backbone, F. Eckstein, H. Gindl, Eur. J. Biochem, 13, 558-564, 1970
Non Patent Literature 2: Phosphorothioate-containing RNAs show mRNA activity in the prokaryotic translation systems in vitro, T. Ueda, H. Tohda, N. Chikazumi, F. Eckstein, K. Watanabe, Nucleic Acids Res. 19 (3), 547-552, 1991 Non Patent Literature 3: Efficient expression of E. coli dihydrofolate reductase gene by an in vitro translation system using phosphorothioate mRNA, H. Tohda, N. Chikazumi, T. Ueda, K. Nishikawa, K. Watanabe, J. Biotechnol, 34, 61-69, 1994

### Summary of Invention

### Technical Problem

Conventional PS-mRNA may have high or low translation efficiency depending on the kind of NTPαS, the number of modifications, or the like, and it has been unclear what kind of PS modification should be applied to mRNA to increase the translation efficiency. Note that Non Patent Literatures 1 to 3 do not disclose the introduction of PS into an untranslated region.

An object of the present invention is to provide an mRNA that is used for synthesis of a protein and has high translation efficiency by ribosome, and a method for producing the mRNA. Further, another object of the present invention is to provide a device for producing a protein and a method for producing a protein, which improve the translation efficiency by using the mRNA.

### Solution to Problem

The present inventors have conducted the intensive studies to solve the above problems. As a result, it has been found that the amount of protein synthesis of the mRNA in which PS has been partially introduced in from the 5' end to the vicinity of the start codon increases 22-fold, but the amount of the synthesis of the mRNA in which PS has been introduced into 250 bases from the vicinity of the start codon to the 3' end decreases by 20%. From these findings, the present inventors have found that the translation efficiency can be significantly improved by introducing PS in from the 5' end to the vicinity of the start codon of mRNA, and thus have completed the present invention.

That is, the present invention is an mRNA for use in synthesis of a protein, including: a translation region containing a start codon and a stop codon; and an untranslated region positioned on the 5'-end side of the start codon, some of phosphate groups within the range of at least from the 5' end of the untranslated region to 15 nt on the 3'-end side of the start codon being substituted with phosphorothioate groups.

In this case, it is preferable that the phosphate groups at two or more positions are the phosphorothioate groups.

Further, in this case, it is preferable that at least some of the phosphate groups in the untranslated region and at least some of the phosphate groups in the translation region are the phosphorothioate groups.

Alternatively, further, it is preferable that two or more kinds of nucleotides of adenosine monophosphate, guanosine monophosphate, cytidine monophosphate, and uridine monophosphate, which form an mRNA, are phosphorothioate groups.

In any of the above cases, it is preferable that the untranslated region contains a Shine-Dalgarno sequence.

In this case, it is preferable that phosphate groups at ten or more positions within the range of at least from the 5' end of the untranslated region to 15 nt on the 3'-end side of the start codon are substituted with phosphorothioate groups, and the phosphorothioate group is contained in both of the Shine-Dalgarno sequence and the translation region.

The present invention is a method for producing the mRNA described in any one of the above, including: a step of preparing a DNA template complementary to the mRNA containing the untranslated region and the translation region; a step of preparing an unmodified NTP containing adenosine triphosphate, guanosine triphosphate, cytidine triphosphate, and uridine triphosphate, and a modified NTP in which at least one kind of phosphate group of the unmodified NTP is substituted with a phosphorothioate group; and a step of performing transcription reaction with RNA polymerase using the DNA template as a template and the unmodified NTP and the modified NTP as substrates.

In this case, it is preferable to include: a DNA cleavage step of cleaving one position in a sequence corresponding to the range of from the 5' end of the untranslated region to 15 nt on the 3'-end side of the start codon in the DNA template to obtain a DNA fragment on the 5'-end side and a DNA fragment on the 3'-end side; a modified mRNA adjustment step of obtaining a modified mRNA fragment on the 5'-end side by performing transcription reaction using the DNA fragment on the 5'end side as a template and the unmodified NTP and the modified NTP as substrates; an unmodified mRNA adjustment step of obtaining an unmodified mRNA fragment on the 3'end side by performing transcription reaction using the DNA fragment on the 3'-end side as a template and only the unmodified NTP as a substrate; and a ligation step of ligating the modified mRNA fragment on the 5'-end side with the unmodified mRNA fragment on the 3'-end side.

Further, the present invention is a device for producing a protein, including: the mRNA described in any one of the above, having a sequence encoding a target protein in the translation region; and a translation system containing at least a ribosome, an NTP, a tRNA, an amino acid, and an aminoacyl-tRNA synthetase.

In the above, as the translation system, a reconstitution E. coli translation system can be mentioned.

In this case, it is preferable that some of phosphate groups of at least one kind of cytidine monophosphate and uridine monophosphate, which form the mRNA, are phosphorothioate groups.

Alternatively, the present invention is a method for producing a protein, including: an mRNA adjustment step of preparing the mRNA described in any one of the above, having a sequence encoding a target protein in the translation region; and a translation step of translating the target protein by a ribosome by adding the mRNA to a translation system containing at least the ribosome, an NTP, a tRNA, an amino acid, and an aminoacyl-tRNA synthetase.

In the above, as the translation system, a reconstitution E. coli translation system can be mentioned.

In this case, it is preferable that some of phosphate groups of at least one kind of cytidine monophosphate and uridine monophosphate, which form the mRNA, are phosphorothioate groups.

### Advantageous Effects of Invention

According to the present invention, an mRNA that is used for synthesis of a protein and has high translation efficiency by ribosome, and a method for producing the mRNA can be provided. Further, as another object of the present invention, a device for producing a protein and a method for producing a protein, which improve the translation efficiency by using the mRNA, can be provided.

### Brief Description of Drawings

Fig. 1 is a diagram showing phosphorothioate modification of the mRNA of the present invention.
Fig. 2 is a diagram showing the effects of PS modification on mRNA translation in a prokaryotic system.
Fig. 3 is a diagram showing the results of reversed-phase high-performance liquid chromatography (HPLC) analysis of NTPaSs used in Examples.
Fig. 4 is a diagram showing the results of Western blot analysis of translation of phosphorothioated mRNA in a protein synthesis using recombinant elements (PURE) system.
Fig. 5 is a graph showing the stability of phosphorothioated mRNA in a PURE translation mixture.
Fig. 6 is a diagram showing the results of single-turnover analysis of the translation reaction of FLAG-EGF-Pro3 mRNA in a PURE system.
Fig. 7 is a diagram showing a difference in the position of translation activity of PS-modified RNA.
Fig. 8 is a diagram showing the results of synthesis experiments of chimeric RNAs containing different PS modification patterns.
Fig. 9 is a diagram showing the results of fluorescence to temperature plotting of RiboGreen reagent mixed with FLAG-EGF mRNA.
Fig. 10 is a diagram showing the results of binding reaction of transcription/translation in a PURE system using NTPaSs.

### Description of Embodiments

### 1. mRNA

Hereinafter, the mRNA according to the present invention will be described. The mRNA according to the present invention is used for synthesis of a target protein, and contains a translation region and an untranslated region. The translation region contains a start codon and a stop codon, and is a region encoding a target protein (coding region). The untranslated region is positioned on the 5'-end side of the start codon, and is a region that is not translated into a protein (noncoding region). This untranslated region contains a sequence to which a ribosome binds (a so-called "consensus sequence"), and as such a sequence, a Shine-Dalgarno sequence (SD sequence) can be mentioned in a prokaryote. In this regard, another untranslated region may be provided downstream on the 3'-end side of the stop codon as long as it does not inhibit the effects of the present invention.

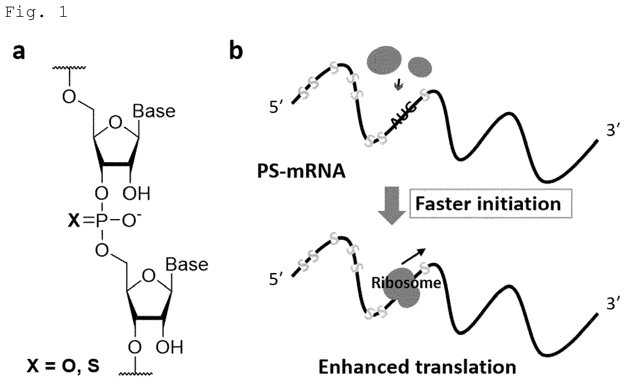

In the mRNA according to the present invention, some of phosphate groups within the range of at least from the 5' end of the untranslated region to 15 nt (nucleotide: base) on the 3'-end side of the start codon (hereinafter, may be referred to as "PS modification range") are substituted with phosphorothioate groups. The expression "15 nt" means the number of nucleotides counted from the nucleotide on the 5' side of the triplet of the start codon (for example, "A" if the start codon is "AUG"). The "phosphate group" referred to herein is a phosphate group of a nucleotide that is a unit forming mRNA, and the phosphorothioate group is a group in which oxygen that doubly bonds to phosphorus of this phosphate group is substituted with sulfur. (a) of Fig. 1 shows a chemical structure of RNA, and indicates a phosphate group (unmodified nucleotide, that is, natural nucleotide) in a case where X is O, and a phosphorothioate group (modified nucleotide) in a case where X is S.

(b) of Fig. 1 is a schematic diagram conceptually showing a mechanism by which the translation efficiency is improved with the use of the mRNA according to the present invention. The upper part of the diagram shows a state before the ribosome binds to mRNA. As shown in this diagram, the mRNA substituted with a phosphorothioate group (PS-mRNA) is PS modified at multiple positions in the PS modification range. A ribosome binds to a consensus sequence (such as a SD sequence) in an untranslated region of the mRNA to form a complex, and the translation is initiated. At this moment, PS-modified mRNA (PS-mRNA) in the PS modification range has a translation initiation rate faster than that of unmodified mRNA (natural mRNA), and the translation efficiency is improved.

As shown in Examples to be described later, as a result of single-turnover analysis of arresting the ribosome during the translation reaction, the rate of peptide elongation reaction by ribosome of PS-mRNA was the same as that of natural mRNA. In addition, it has been revealed that the translation initiation rate (rate of ribosome complex formation) is faster in the PS-mRNA template than that in natural one. In general, since the rate-limiting step is considered to be in the translation initiation stage during translation cycle, it is considered that the high translation efficiency of PS-mRNA is due to the acceleration of process of ribosome complex formation by the PS-mRNA. This result brings about a new strategy for improving the translation ability by using non-natural mRNA.

Further, as shown in Examples to be described later, the amount of synthesis of the mRNA in which PS had been partially introduced from the 5' end to the translation region in the vicinity of the start codon (34 bases from the 5' end = 11 bases from of the start codon) increased 22-fold. Meanwhile, the amount of synthesis of the mRNA in which PS has been introduced into 250 bases from the vicinity of the start codon to the 3' end decreased by 20%. From these results, in view of the translation efficiency, the site to which PS is introduced is not any site in the mRNA, but is limited to from the 5'end to the translation region in the vicinity of the start codon (20 bases on the downstream side from the start codon).

In the mRNA according to the present invention, phosphate groups at one or more positions are substituted with phosphorothioate groups, and it is preferable that phosphate groups at two or more positions are phosphorothioate groups. If there are few substitution positions, the translation efficiency tends to be lowed. The number of substitution positions of PS is preferably five or more, and more preferably ten or more. The upper limit of the number of substitution positions is not particularly limited, and is preferably 20 or less.

Further, in the PS modification range, it is preferable that at least some of the phosphate groups in the untranslated region and at least some of the phosphate groups in the translation region are both substituted with phosphorothioate groups. In addition, in the present invention, it is acceptable that at least the PS modification range is PS modified, and a translation region on the 3'-end side of the PS translation region, and also an untranslated region in a case where there is another untranslated region on the 3'-end side of the stop codon, may be PS modified. However, as shown in Examples to be described later, since the translation efficiency is higher in a case where the PS modification is only in the PS modification range than in a case where the PS modification is also in a range other than the PS modification range, it is preferable that only the PS modification range is PS modified.

In the mRNA according to the present invention, only one kind of adenosine monophosphate (AMP), guanosine monophosphate (GMP), cytidine monophosphate (CMP), and uridine monophosphate (UMP), which form the mRNA, is a phosphorothioate group. Alternatively, among these nucleotides, two or more kinds of nucleotides may be phosphorothioate groups. Because of depending on the secondary structure or the like of mRNA, it is not unconditionally determined which nucleotide of these nucleotides is PS modified to improve the translation efficiency, and therefore, it is better to design appropriately according to the sequence of the unmodified region, the translation system, or the like.

### 2. Method for producing mRNA

Next, a method for producing an mRNA will be described. In a method for producing an mRNA, first, a DNA template complementary to the mRNA containing an untranslated region and a translation region is prepared (DNA adjustment step). In the DNA template, the part complementary to the untranslated region of the mRNA includes a promoter sequence, a sequence complementary to the above-described SD sequence, and the like.

Further, an (unmodified) NTP containing adenosine triphosphate (ATP), guanosine triphosphate (GTP), cytidine triphosphate (CTP), and uridine triphosphate (UTP) is prepared. Furthermore, a modified NTP (NTPaSs) containing modified adenosine triphosphate (ATPαSs), modified guanosine triphosphate (GTPαSs), modified cytidine triphosphate (CTPαSs), and modified uridine triphosphat (UTPαSs), in which a phosphate group of NTP is substituted with a phosphorothioate group, is prepared. A reagent containing these NTP and NTPaSs is prepared. Subsequently, transcription reaction is performed by RNA polymerase by using the above DNA template as a template and the NTP and NTPaSs contained in the reagent as substrates. As the RNA polymerase, T7 RNA polymerase or the like can be mentioned. The ratio of NTP to NTPaSs may be appropriately set within the range of 1 : 100 to 100 : 1 although it depends on the number of PSs to be introduced into the mRNA. The reaction temperature and reaction time of the transcription reaction may also be appropriately set, and for example, the reaction temperature may be 20 to 50°C, and the reaction time may be 30 minutes to 6 hours.

Next, a method for producing a PS-mRNA in which only the PS modification range is PS modified (mRNA having no PS modification in any range except for the PS modification range) will be described.

First, in the above DNA template, one position in a sequence corresponding to the range (PS modification range) from the 5' end of the untranslated region to 15 nt on the 3'-end side of the start codon is cleaved. In this way, a DNA fragment on the 5'-end side and a DNA fragment on the 3'-end side are obtained (DNA cleavage step). For the cleavage of the DNA template, a restriction enzyme or the like is used.

Next, the transcription reaction is performed by using the DNA fragment on the 5'-end side as a template, and both of the NTP and the NTPaSs as substrates to obtain a modified mRNA fragment on the 5'-end side (modified mRNA adjustment step). Further, the transcription reaction is performed by using the DNA fragment on the 3'-end side as a template, and only the NTPaSs as a substrate to obtain an unmodified mRNA fragment on the 3'-end side (unmodified mRNA adjustment step). In the step of obtaining these mRNA fragments, such mRNA fragments can be obtained by using T7 RNA polymerase or the like and applying the above reaction conditions.

Finally, the modified mRNA fragment on the 5'-end side is ligated with the unmodified mRNA fragment on the 3'-end side (ligation step). This step can be performed by using an enzyme such as T4 RNA ligase. In this way, a chimeric mRNA in which the modified mRNA fragment on the 5'-end side is bound to the unmodified modified mRNA fragment on the 3'-end side can be produced.

### 3. Device and method for producing protein

Next, the device for and the method for producing a protein according to the present invention will be described. The mRNA according to the present invention can be used for producing a target protein. First, an mRNA having a sequence encoding a target protein in the translation region is prepared (mRNA adjustment step). The mRNA can be adjusted by using one containing a sequence encoding a target protein in the above DNA template.

Next, the translation is performed by adding the mRNA to a translation system (translation step). As the translation system, a system containing at least a ribosome, an NTP, a tRNA, an amino acid, and an aminoacyl-tRNA synthetase is used. The translation system is preferably a reconstitution E. coli translation system (a so-called PURE system). The PURE system is a system obtained by reconstituting the translation system of E. coli in vitro. The PURE system contains an initiation factor (IF1, IF2, IF3), an elongation factor (EF-Tu, EF-Ts, EF-G), a termination (dissociation) factor (RF1, RF2, RF3), a ribosome regeneration factor (RRF), an aminoacyl-tRNA synthetase (ARS), a methionyl-tRNA formyltransferase, a ribosome, a T7 RNA polymerase, an amino acid, an NTP, a tRNA, and the like. By adding an mRNA to the PURE system and performing the translation, a target protein can be obtained. The reaction temperature and reaction time may be appropriately set, and for example, the reaction temperature may be 20 to 50°C, and the reaction time may be 1 to 24 hours. In the PURE system, since ATP and GTP as the substrates are easily decomposed in the system, it is preferable to use CTPαSs and/or UTPaSs.

### Examples

Hereinafter, the present invention will be specifically described by way of Examples, but these Examples do not limit the object of the present invention. Further, in the following Examples, the expression "%" is on a mass basis (mass percent) unless otherwise particularly specified.

### 1. Experimental Example 1: Sequence design, synthesis of PS-mRNA, and synthesis of protein (FLAG-EGF) using E. coli cell-free translation system

The upper part of Fig. 2(a) shows a schematic diagram of transcription of FLAG-EGF mRNA from the corresponding dsDNA as a template. In this experiment, at first, an mRNA FLAG-EGF containing a Shine-Dalgarno (SD) sequence, three FLAG octapeptide coding sequences, and an epidermal growth factor (EGF) coding sequence was designed as a model sequence.

The FLAG-EGF mRNA was prepared by in vitro transcription reaction using T7 RNA polymerase and a dsDNA template obtained by polymerase chain reaction (PCR) amplification. The PS-mRNA was prepared by substituting one or multiple NTPs with the corresponding NTPαS. PS-mRNAs having possible 16 combinations of unmodified NTP and NTPαS were synthesized.

The lower part of Fig. 2(a) shows the analysis results of denaturing PAGE of in vitro transcription by T7 RNA polymerase using NTPaSs. One or more NTPs are substituted with the corresponding NTPaSs, and are represented by the symbol "+" in the diagram. The gel was visualized by SYBR Green II staining. Transcription reactions were performed in all the 16 combinations, and the transcripts were analyzed by denaturing polyacrylamide gel electrophoresis (PAGE). The gel was stained and the relative amount of transcription RNA was calculated.

As a result, it was found that the target RNA was observed in all the combinations, and the relative yield of mRNA decreased as the number of substituted NTPs increased.

Next, translation reactions of 16 kinds of FLAG-EGF mRNAs having different PS modification patterns were compared at the same concentration in a PURE system. As the PURE system, a system provided by Dr. Yoshihiro Shimizu of RIKEN was used. The preparation method is disclosed in the following papers and the like.

Cell-free translation reconstituted with purified components, Y. Shimizu, A. Inoue, Y. Tomari, T. Suzuki, T. Yokogawa, K. Nishikawa, T. Ueda, Nat. Biotechnol. 2001, 19, 751-755; and b) The PURE System for Protein Production, Y. Shimizu, Y. Kuruma, T. Kanamori, T. Ueda, in Cell-Free Protein Synthesis: Methods and Protocols, Vol. 1118 (Eds.: K. Alexandrov, W. A. Johnston), Springer Nature Switzerland AG, 2014, pp. 275-284.

Fig. 2(b) is a diagram showing the results of Western blot analysis after translation of FLAG-EGF mRNA in a PURE system. After transcription, purified RNA (0.2 µM) was incubated at 37°C for 2 hours in a system. The modification pattern of RNA during the transcription was represented by the symbol "+" indicating the substituted NTPaSs. The translation product was detected by using an anti-FLAG M2 antibody. The relative amounts of the translation product obtained from PS-mRNA and the translation product obtained from PO-mRNA were calculated by a Western blot method.

As a result, all the PS-mRNAs showed higher translation amounts than that of PO-mRNA. The highest relative ratio was 11 times when both UTPαS and CTPαS were used, and the second highest relative ratio was 8.6 times when only ATPαS was used.

Next, an isomer contained in NTPαS was analyzed. Fig. 3 is a diagram showing the results of reversed-phase high-performance liquid chromatography (HPLC) analysis of NTPaSs used in this experiment. As a result of the HPLC analysis, the NTPαS purchased from TriLink BioTechnologies was an equivalent mixture of Rp and Sp isomers. In this regard, it has been indicated that T7 RNA polymerase uses only Sp type as a substrate and Rp type does not inhibit the transcription reaction.

### 2. Experimental Example 2: Synthesis of protein by other sequences (AcGFP, and Spider-FLAG)

Next, in order to determine whether there is any generality in the improvement of translation efficiency by PS modification, it was examined by using two other sequences (AcGFP, and Spider-FLAG), which are different from the FLAG-EGF of Experimental Example 1.

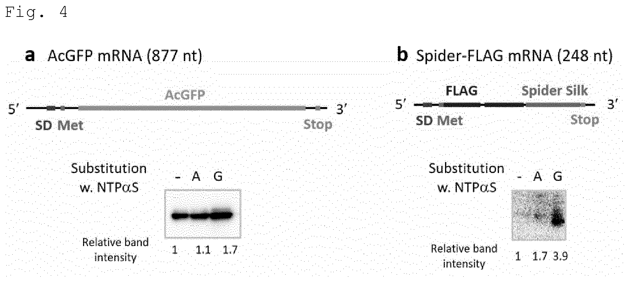

Fig. 4 is a diagram showing the results of Western blot analysis of translation of phosphorothioated mRNA in a PURE system. AcGFP mRNA (a) and Spider-FLAG mRNA (b) were transcribed by the substitution of ATP or GTP with ATPαSs or GTPαSs, respectively. After the transcription, purified RNA (0.2 µM) was incubated at 37°C for 2 hours in the PURE system. The translation product was detected by using an anti-GFP antibody (Santa Cruz Biotech, sc-9996) for the AcGFP mRNA and an anti-FLAG M2 antibody (Sigma-Aldrich, F1804) for the Spider-FLAG mRNA.

Eventually, ATPαS or GTPαS was introduced into the Spider-FLAG mRNA and the AcGFP mRNA, and the translation amount of PS-mRNA was compared with that of PO-mRNA. As a result, the translation amount of each PS-mRNA was improved as compared with that of PO-mRNA. Further, the relationship between the PS introduction position of EGF-FLAG mRNA and the translation amount was analyzed by bioinformatics, but the relationship was not able to be found. The distribution of PS in the mRNA, rather than the kind of base to be substituted, may be a factor in determining the translation ability.

### 3. Experimental Example 3: Stability of phosphorothioated mRNA in PURE translation mixture

Next, it was investigated why the introduction of PS into the mRNA improved the translation efficiency. As one of the reasons, there is a possibility that the improvement is due to the stability of PS-mRNA. PS(A,C)-mRNA that is a FLAG-EGF mRNA transcribed by the substitution of ATP and CTP with ATPαS and CTPaS, respectively, was incubated in a translation liquid, and then the stability of the PS(A,C)-mRNA was compared with that of PO-mRNA by quantifying the mRNA by RT-PCR.

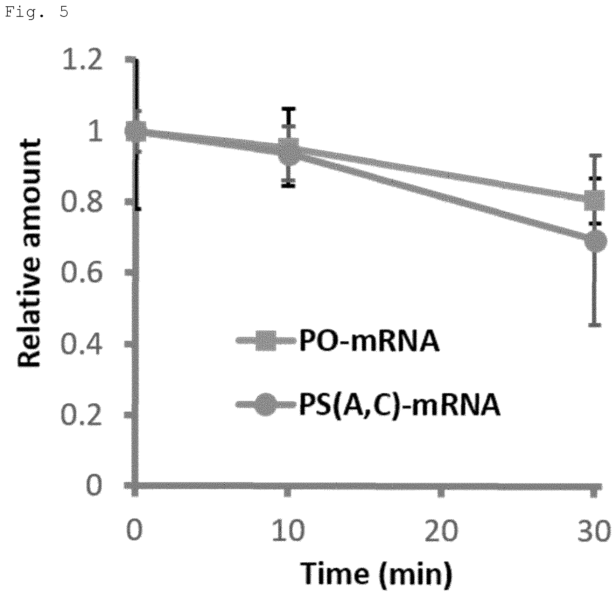

Fig. 5 is a graph showing the stability of phosphorothioated mRNA in a PURE translation mixture [50 mM HEPES-KOH (pH 7.6), 100 mM potassium glutamate, 2 mM spermidine, 1 mM DTT, 20 mM sodium creatine phosphate, and 0.1 mM 20 amino acid each]. PO-mRNA and PS(A,C)-mRNA of the FLAG-EGF sequence were incubated at 37°C and a concentration of 0.05 pmol/µL. After 10 minutes and 30 minutes, an aliquot (2 µL) was taken from the mixture, and mixed with 148 µL of water, and the obtained mixture was stored at -30°C. The concentration of RNA in these solutions was determined by quantitative reverse transcription-polymerase chain reaction (qRT-PCR). The qRT-PCR was performed by a CFX Connect real-time PCR detection system (Bio-Rad) using One Step TBGreen (registered trademark) PrimeScript (trademark) RT-PCR Kit (Takara Bio).

The primer sequences used were FLAG-EGF_F: 5'-CAATATACATGCACACACCG-3', and FLAG-EGF_R: 5'-GGGATCCGAAGGAGATATATCC-3'. Renilla luciferase mRNA (prepared by using pRL-TK of BioRad) was used as an internal control of the qRT-PCR by using a primer set (Rluc_F: 5'-GTTGGACGACGAACTTCACC-3', and Rluc_R: 5'-ATCATGGGATGAATGGCCTG-3'). Reaction conditions: 42°C for 5 minutes → 95°C for 10 seconds → (95°C for 5 seconds → 60°C for 30 seconds) × 40 cycles.

As a result, no difference was observed in the stability between the two mRNAs. Since the PURE system is a reconstitution of an E. coli translation system and does not contain any nuclease, it is considered that RNA degradation reaction is less likely to occur. Accordingly, it was determined that the stability of PS-mRNA was not the main factor in improving the translation efficiency.

### 4. Experimental Example 4: Single-turnover analysis of translation reaction of FLAG-EGF-Pro3 mRNA in PURE system

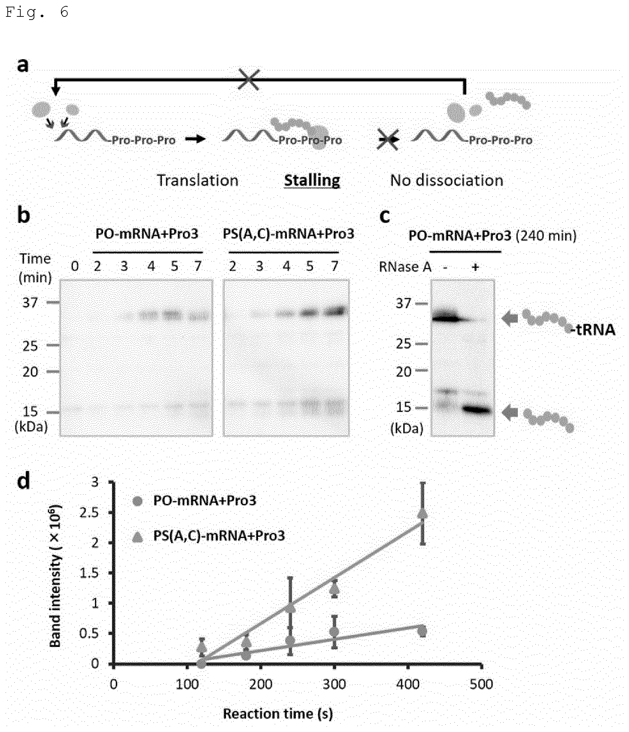

Next, attention was paid to the mechanism of translation reaction. The translation reaction of ribosome is roughly classified into three stages of initiation, elongation, and termination. Among them, the initiation stage, that is, a stage in which the ribosome binds to mRNA takes the longest time, and is considered to be a rate-limiting step. For this reason, it was hypothesized that the introduction of PS into the mRNA accelerated the binding rate of ribosome, and as a result of which the translation efficiency was improved. In order to verify the hypothesis, the initial rate of translation reaction was analyzed. In order to capture the initial rate of translation reaction, experimental conditions for a single turnover in which the ribosome does not dissociate were set (Fig. 6(a)). Fig. 6 is a diagram showing the results of single-turnover analysis of the translation reaction of FLAG-EGF-Pro3 mRNA in a PURE system, and Fig. 6(a) shows an outline of the experimental design.

So far, it has been reported that by the introduction of three or more proline (Pro) repeat sequences at the end of a protein sequence, the ribosome is arrested without the dissociation at the site. In view of this, a FLAG-EGF-Pro3 mRNA in which three Pro sequences were introduced at the end of the FLAG-EGF mRNA was designed and synthesized, and the translation reaction was performed. PS(A,C)-mRNA was prepared by transcription with the use of a combination of ATPαS and CTPαS of which the translation amount was improved in the previous experimental results. The translation reaction was performed for 2 to 7 minutes, and then the amount of protein synthesis was analyzed by a Western blot method (Fig. 6).

Fig. 6(b) shows the time course of translation reaction of phosphorothioated mRNA (PS(A,C)-mRNA+Pro3) or unmodified mRNA (PO-mRNA+Pro3) analyzed by Western blotting.

In the translation reaction of FLAG-EGF mRNA, a band of the product was generated at the position of about 15 kDa, but in the translation reaction of FLAG-EGF-Pro3 mRNA, a band of the product was generated in the vicinity of 37 kDa (Fig. 6(b)). This is because the ribosome cannot perform peptide elongation on a Pro repeat and the termination reaction does not occur, so that the reaction is terminated in a state that tRNA is bound, and the tRNA is obtained as the translation product.

Fig. 6(c) shows the results of Western blot analysis of the translation product after RNase A treatment. The mRNA (PO-mRNA+Pro3) was translated at 37°C for 240 minutes in a system, and then RNase was added to the mixture.

As a result, when the translation product was actually treated with RNase A that is a ribonuclease, tRNA was degraded and a product was observed in the vicinity of 15 kDa. This result supports that this system can analyze the single-turnover reaction of ribosome.

Fig. 6(d) shows reaction time converted plotting. The signal intensities of bands that appeared between the 25-kDa marker and the 37-kDa marker were plotted.

As a result, when the time changes of the amounts of the products in the vicinity of 37 kDa generated from a natural mRNA and a PS-mRNA were plotted, the x-intercepts of the two straight lines showed almost the same values of around 100 seconds. This indicates that the elongation rates of the natural mRNA and PS-mRNA in the translation reaction are almost the same. The slope of each graph represents the initial rate of translation reaction, and this was found to be around 4 times faster for the PS-mRNA than for the PO-mRNA. Since the rate-limiting step of translation reaction is in the initiation stage (binding of ribosome), the PS modification promotes the binding of ribosome to mRNA, and it was indicated that the promotion is the main factor in improving the translation activity.

### 5. Example and Comparative Example: Effect of difference in PS modification introduction position on translation reaction

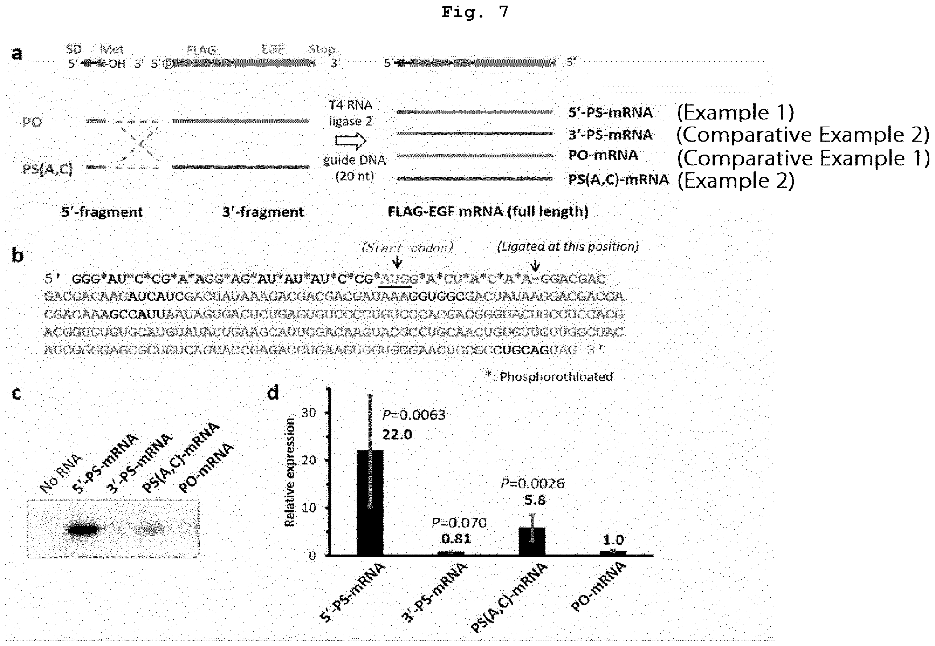

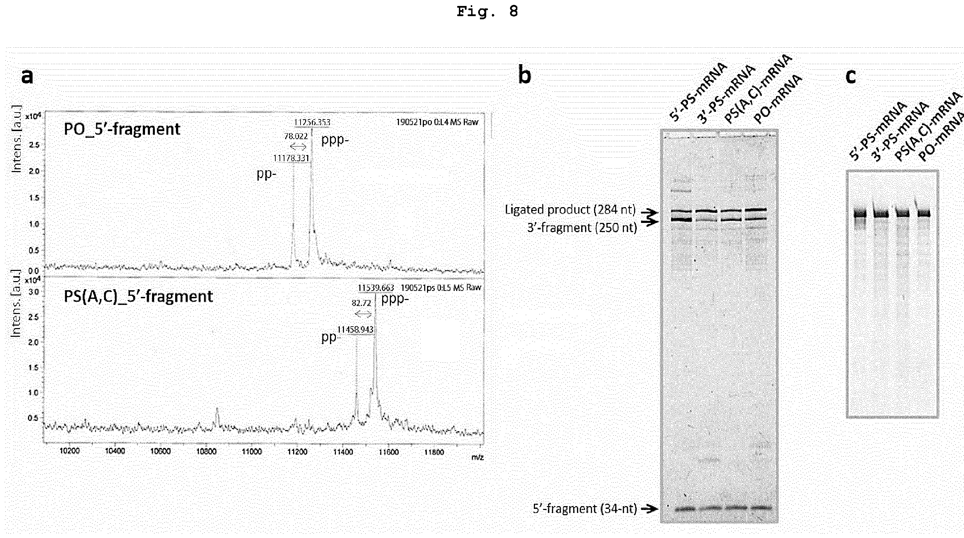

Next, two chimeric mRNAs were designed on the basis of the FLAG-EGF sequence. For the 5'-PS-mRNA, PS was introduced into A and C bases of 34 nt (11 nt from the start codon) from the 5' end, which mainly formed of an UTR (untranslated region). For the 3'-PS-mRNA, PS was introduced into A and C bases in 250 bases from the 3' end (Figs. 7 and 8). These were obtained by ligating two RNA strands with the use of T4 RNA ligase 2 (Fig. 8).

Fig. 7 is a diagram showing a difference in the position of translation activity of the PS-modified RNA. (a) of the diagram shows synthetic schemes of FLAG-EGF mRNAs having different PS modification patterns. (b) of the diagram shows a nucleotide sequence of 5'-PS-mRNA. (c) of the diagram shows the results of Western blot analysis of four mRNAs in a PURE system. (d) of the diagram shows the quantitative results of the relative expression levels from the four RNAs based on the Western blotting shown in (c). The error bar represents the standard deviation (n = 3). The P values were calculated on the basis of an unpaired t-test.

Fig. 8 is a diagram showing the results of synthesis experiments of chimeric RNAs containing different PS modification patterns. (a) of the diagram shows the results of MALDI-TOF MS analysis of 34 nt RNA 5'-fragment. The main peak corresponds to a 5'-triphosphorylated 34 nt transcript, and the minor peak corresponds to a 5'-pyrophosphorylated transcript. (b) of the diagram shows the analysis results of 6% denaturing PAGE of ligation reaction for preparing chimeric RNA. (c) of the diagram shows the analysis results of RNA after purification by preparative PAGE. The gel was visualized by SYBR Green II staining. In (c), each RNA of 0.5 pmol was analyzed.

As a result of evaluation of translation activity of these chimeric mRNAs, when compared with the PO-mRNA without PS modification (Comparative Example 1), the 5'-PS-mRNA (Example 1) showed a large increase of 22 times, the 3'-PS-mRNA (Comparative Example 2) showed a decrease of 0.8 times, and the PS(A,C)-mRNA (Example 2) in which PS modification was applied to the entire mRNA showed an increase increase of 5.8 times (Figs. 7(c) and 7(d)). The results mean that the introduction of PS in from the 5' end to the vicinity of the start codon is a main factor in increasing the translation amount. Further, when considering that the translation amount of the PS(A,C)-mRNA is lower than that of the 5'-PS-mRNA, it is suggested that the introduction of PS in from the start codon to the 3' end exerts an effect of decreasing the translation amount. When making a determination from the results obtained so far, it is determined that the introduction of PS in from the 5' end to the vicinity of the start codon promotes the initiation of translation and increases the amount of translation product.

### 5. Experimental Example 5: Evaluation of duplex-forming ability and thermodynamic stability of PO-mRNA and PS(A,C)-mRNA

It is known that the formation of secondary structure of mRNA influences the ribosome complex formation in the translation initiation stage. The results shown in Figs. 6 and 7 suggest a possibility of the contribution by the decrease in the secondary structure formation around the 5' UTR region due to the introduction of PS modification. In a case where there are less secondary structures in the mRNA sequence from the 5' UTR to the start codon, the translation initiation is faster. However, it has been reported that (Rp)-PS-RNA forms a thermodynamically more stable double strand with PO-RNA. By using RiboGreen reagent that is known to fluoresce at the site of double-strand formation in RNA, the duplex-forming abilities and thermodynamic stabilities of PO-mRNA and PS(A,C)-mRNA were evaluated.

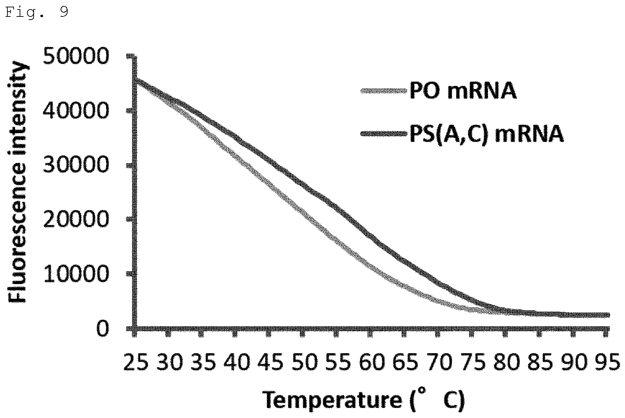

Fig. 9 is a diagram showing the results of fluorescence to temperature plotting of RiboGreen reagent mixed with FLAG-EGF mRNA. RNA [0.2 µM, PO-mRNA or PS(A,C)-mRNA] was mixed with a PURE system buffer [50 mM HEPES-KOH (pH 7.6), 100 mM potassium glutamate, 2 mM spermidine, 13 mM Mg(OAc)₂, 1 mM DTT, 20 mM sodium creatine phosphate, 0.1 mM 20 amino acid each, 2 mM ATP, 2 mM GTP, 1 mM CTP, and 1 mM UTP] together with Quant-iT RiboGreen RNA Reagent (400-fold dilution, Thermo Fisher Scientific). The RNA in solution (25 µL) was annealed by lowering the temperature from 95°C to 25°C, and then fluorescence (Ex 450 to 490 nm, Em 515 to 530 nm) was recorded every 5°C from 25 to 95°C with a CFX Connect real-time PCR detection system (Bio-Rad) while increasing the temperature from 25°C.

As a result, it was found that the secondary structure of PS(A,C)-mRNA is slightly more stable than that of PO-mRNA. The physical properties of these mRNAs cannot explain the increased translation efficiency in terms of RNA higher-order structure formation. Other RNA higher-order structure formations or other factors may be involved in the promotion of a ribosome initiation complex, and which must be elucidated in the future.

### 6. Experimental Example 5: Coupled reaction between transcription and translation using NTPαS

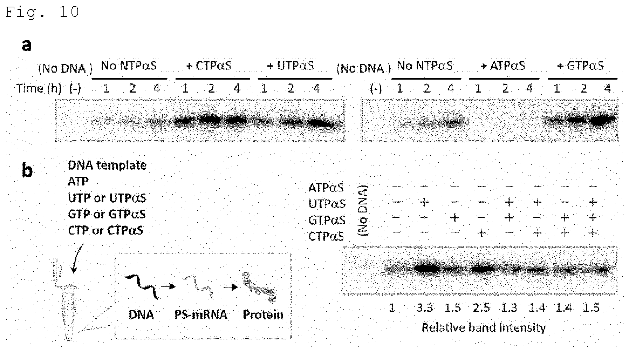

In the experiments so far, the transcribed PS-mRNA was isolated and purified before being used for translation reaction, but in consideration of the practicality, it was decided to investigate whether the addition of NTPαS to the transcription/translation coupled system can also improve the translation efficiency. However, ATP and GTP are substrates for aminoacylation reaction, or a GTPase that functions in a translation system such as initiation factor 2 or elongation factor G. For this reason, substitution with ATPαS or GTPαS was expected to influence the translation reaction. First, by using the template DNA shown in Fig. 2a, the transcription/translation coupled reaction in which one base of NTP was substituted with NTPαS was performed in a PURE system. The obtained translation product was analyzed by Western blotting (Fig. 10(a)).

Fig. 10 is a diagram showing binding reaction of transcription/translation in a PURE system using NTPaSs. (a) of the diagram shows the results of Western blot analysis of translation products using template DNA encoding FLAG-EGF mRNA with one NTPaSs substituted. The substituted NTPaSs are shown in the diagram. The left side of (b) of the diagram shows a strategy for enhancing the translation by using NTPaSs in the PURE system. With the exception of ATPaSs, one or more NTPs were replaced with the corresponding NTPaSs. The right side of (b) shows the results of Western blot analysis of the reaction after 2 hours of incubation with one or more NTPaSs substitutions except for ATPaSs, using template DNA encoding a FLAG-EGF mRNA.

As a result, in a case of being substituted with ATPαS, UTPαS, and GTPαS, a translation product with a level higher than that of the reaction of unsubstitution was given, but in a case of being substituted with ATPαS, no translation product was generated. ATP is hydrolyzed to AMP by an aminoacyl-tRNA synthetase in the PURE system. Accordingly, the possibility is suggested that the enzyme reaction was suppressed due to the substitution with ATPαS, and as a result of which all the translation reactions were suppressed. In contrast, GTP was hydrolyzed to GDP by some GTPases in the translation reaction, but substitution with GTPαS did not influence the translation efficiency, and it has been considered that GTPαS played a role equivalent to that of GTP. Therefore, the different effects of ATP and GTP substitutions on the translation efficiency may have been caused by the difference between the hydrolysis products (AMP and GDP).

Further, the present inventors analyzed the effect of substitution with two or more NTPαS by using CTPαS, UTPαS, and GTPαS (Fig. 10b). When compared with the results using the unmodified NTP, a larger amount of translation products were given in all of the substitution combinations. In particular, substitution with UTPαS or CTPαS was the most effective, and the translation product around 3 times was given in such a substitution. The technique using NTPαS is a useful technique because the translation amount can be increased only by the substitution with NTP in a cell-free protein translation system.

As in the above, in the present Examples, it has been indicated that the introduction of PS into mRNA improves the amount of protein synthesis in an E. coli cell-free translation system up to 22 times. It has been suggested that the introduction of PS increases the binding rate of ribosome which is an initiation stage of the translation reaction. Further, it has been revealed that the mRNA in which PS was introduced in from the 5' end to the vicinity of the start codon is the most suitable molecular design, and is important for accelerating the initiation stage. Furthermore, as a result of simultaneous reactions of transcription and translation by using NTPαS, it has been revealed that a higher translation amount is given as compared with the case of using unmodified NTP. The present technique can be a useful technique in an E. coli cell-free translation system.

The mRNA according to the present invention can be applied not only to the translation system of a prokaryote but also to the translation system of a eukaryote, and can improve the translation efficiency of a target protein. Further, the mRNA according to the present invention can efficiently translate a target protein, and therefore, is useful not only for industrial production of protein but also for mRNA medicine or the like.

## Claims

1. An mRNA for use in synthesis of a protein, comprising:
a translation region containing a start codon and a stop codon; and
an untranslated region positioned on the 5'-end side of the start codon,
some of phosphate groups within the range of at least from the 5' end of the untranslated region to 15 nt on the 3'-end side of the start codon being substituted with phosphorothioate groups.

2. The mRNA according to claim 1, wherein the phosphate groups at two or more positions are the phosphorothioate groups.

3. The mRNA according to claim 2, wherein at least some of the phosphate groups in the untranslated region and at least some of the phosphate groups in the translation region are the phosphorothioate groups.

4. The mRNA according to claim 1, wherein two or more kinds of nucleotides of adenosine monophosphate, guanosine monophosphate, cytidine monophosphate, and uridine monophosphate, which form an mRNA, are phosphorothioate groups.

5. The mRNA according to claim 1, wherein the untranslated region contains a Shine-Dalgarno sequence.

6. The mRNA according to claim 5, wherein
phosphate groups at ten or more positions within the range of at least from the 5' end of the untranslated region to 15 nt on the 3'-end side of the start codon are substituted with phosphorothioate groups, and
the phosphorothioate group is contained in both of the Shine-Dalgarno sequence and the translation region.

7. A method for producing the mRNA according to any one of claims 1 to 6, comprising:
a step of preparing a DNA template complementary to the mRNA containing the untranslated region and the translation region;
a step of preparing an unmodified NTP containing adenosine triphosphate, guanosine triphosphate, cytidine triphosphate, and uridine triphosphate, and a modified NTP in which at least one kind of phosphate group of the unmodified NTP is substituted with a phosphorothioate group; and
a step of performing transcription reaction with RNA polymerase using the DNA template as a template and the unmodified NTP and the modified NTP as substrates.

8. The method for producing the mRNA, according to claim 7, comprising:
a DNA cleavage step of cleaving one position in a sequence corresponding to the range of from the 5' end of the untranslated region to 15 nt on the 3'-end side of the start codon in the DNA template to obtain a DNA fragment on the 5'-end side and a DNA fragment on the 3'end side;
a modified mRNA adjustment step of obtaining a modified mRNA fragment on the 5'-end side by performing transcription reaction using the DNA fragment on the 5'end side as a template and the unmodified NTP and the modified NTP as substrates;
an unmodified mRNA adjustment step of obtaining an unmodified mRNA fragment on the 3'-end side by performing transcription reaction using the DNA fragment on the 3'end side as a template and only the unmodified NTP as a substrate; and
a ligation step of ligating the modified mRNA fragment on the 5'-end side with the unmodified mRNA fragment on the 3'-end side.

9. A device for producing a protein, comprising:
the mRNA according to any one of claims 1 to 6, having a sequence encoding a target protein in the translation region; and
a translation system containing at least a ribosome, an NTP, a tRNA, an amino acid, and an aminoacyl-tRNA synthetase.

10. The device for producing a protein according to claim 9, wherein the translation system is a reconstitution E. coli translation system.

11. The device for producing a protein according to claim 10, wherein some of phosphate groups of at least one kind of cytidine monophosphate and uridine monophosphate, which form the mRNA, are phosphorothioate groups.

12. A method for producing a protein, comprising:
an mRNA adjustment step of preparing the mRNA according to any one of claims 1 to 6, having a sequence encoding a target protein in the translation region; and
a translation step of translating the target protein by a ribosome by adding the mRNA to a translation system containing at least the ribosome, an NTP, a tRNA, an amino acid, and an aminoacyl-tRNA synthetase.

13. The method for producing a protein according to claim 12, wherein the translation system is a reconstitution E. coli translation system.

14. The method for producing a protein according to claim 13, wherein some of phosphate groups of at least one kind of cytidine monophosphate and uridine monophosphate, which form the mRNA, are phosphorothioate groups.
